# EUROPEAN PATENT SPECIFICATION

(11) **EP 3 431 976 B1**
(45) Date of publication and mention of the grant of the patent: **20.04.2022**
(21) Application number: 18184917.5
(22) Date of filing: 23.07.2018
(51) Int. Cl.: G01N 27/16, G01N 33/00

(54) **GAS DETECTOR COMPRISING SILOXANE REMOVAL FILTER**
GASDETEKTOR MIT EINEM SILOXANEFILTER
DÉTECTEUR DE GAZ COMPRENANT UN FILTRE D'ÉLIMINATION DE SILOXANE

(30) Priority: 21.07.2017 JP 2017141768
(43) Date of publication of application: 23.01.2019
(73) Proprietor: Riken Keiki Co., Ltd., Tokyo 174-8744 (JP)
(72) Inventor: Tanaka, Yuki, Tokyo, 174-8744 (JP); Asada, Ryuji, Tokyo, 174-8744 (JP); Shibasaki, Yoshikazu, Tokyo, 174-8744 (JP); Ono, Kei, Tokyo, 174-8744 (JP)
(74) Representative: Grünecker Patent- und Rechtsanwälte PartG mbB

(56) References cited:
- WO-A1-2018/085026
- JP-A- H10 307 115
- JP-A- 2006 194 851
- JP-A- 2006 234 415
- JP-A- 2007 057 295
- JP-A- 2016 223 925
- US-A1- 2008 226 505
- Gabriela Soreanu ET AL: "Approaches concerning siloxane removal from biogas - A review", Canadian Biosystems Engineering, 1 January 2011 (2011-01-01), page 8_1, XP055516830, Saskatoon Retrieved from the Internet: URL:http://www.csbe-scgab.ca/docs/journal/ 53/C0815.pdf [retrieved on 2018-10-18]
- JAI B. LAD ET AL: "Adsorption of dimethyl ether (DME) on zeolite molecular sieves", CHEMICAL ENGINEERING JOURNAL, vol. 256, 9 July 2014 (2014-07-09), pages 335-346, XP055517609, ISSN: 1385-8947, DOI: 10.1016/j.cej.2014.07.001

## Description

### TECHNICAL FIELD

The present invention relates to a gas detector which is provided with a contact combustion-type gas sensor. The gas detector of the present invention is defined in claim 1. Details of the gas detector are described in the dependent claims.

### BACKGROUND ART

For example, a certain type of contact combustion-type gas sensors used to detect a flammable gas is configured to include a gas detection element with a gas sensitive part firmly fixed to the surface of a temperature-measuring resistor that generates heat when energized. The gas sensitive part is configured such that an oxidation catalyst is carried by a carrier made of a metal oxide sintered compact.

In such a contact combustion-type gas sensor, when a silicone compound or a poisonous substance such as hexamethyldisiloxane or silicone oil exists in the atmosphere of a space to be measured, the silicone compound is adsorbed and accumulated (poisoned) on the surface of the oxidation catalyst. Thus, in such a contact combustion-type gas sensor, the performance (activity) of the oxidation catalyst deteriorates to gradually degrade the detection sensitivity.

In view of such a problem, for example, it is conceivable to dispose a silicone removal filter and thereby prevent the poisoning of the gas detection element. A gas sensor that is provided with such a silicone removal filter is disclosed, for example, in Patent Literature 1. Further sensors are disclosed in Patent Literature 2 to 9. Non-Patent Literature 1 discloses methods for removing siloxane from biogas. Non-Patent Literature 2 discloses zeolite molecular sieves as adsorbents for dimethyl ether.

### CITATION LIST

### PATENT LITERATURE

Patent Literature 1: JP 2006-250569 A
Patent Literature 2: JP 2006-234834 A
Patent Literature 3: JP 2007-057294 A
Patent Literature 4: JP 2006-194851 A
Patent Literature 5: JP 2016-223925 A
Patent Literature 6: WO 2018/085026 A1
Patent Literature 7: JP 2006-234415 A
Patent Literature 8: US 2008/226505 A1
Patent Literature 9: JP H10 307115 A
Non-Patent Literature 1: G. Soreanu et al., Canadian Biosystems Engineering 2011, 53, 8.1-8.18.
Non-Patent Literature 2: J. B. Lad et al., Chemical Engineering Journal 2014, 256, 335-346.

### SUMMARY OF INVENTION

### Technical Problem

However, in a gas sensor as mentioned above, since part of a target gas to be detected, for example, a solvent gas may be removed by the silicone removal filter, the target gas to be detected cannot be properly detected.

Furthermore, in order to prevent the detection sensitivity of the gas detection element from being degraded, it is also conceivable to increase the amount of an oxidation catalyst by increasing the amount of carrier that constitutes the gas sensitive part in the gas detection element. However, in order to heat such a gas detection element to a temperature that is required to provide a sufficient gas sensitivity for the gas detection element, a greater power is required. Thus, a battery-driven portable gas detector is not adequate due to a short service time (operation time).

The present invention has been made in view of the foregoing circumstances, and has as its object the provision of a gas detector which has a high durability to silicone poisoning and of which power consumption is reduced.

A gas detector of the present invention, as defined in claim 1, includes a contact combustion-type gas sensor and an output processing unit.

The contact combustion-type gas sensor includes a case in which two detection chambers (Sa, Sb)are formed that are partitioned by a partitioning plate, and two gas detection elements (20a, 20b) are each disposed in each of two detection chambers that are partitioned from each other, the gas detection elements each having a catalyst carried by a carrier made of a metal oxide sintered compact firmly fixed to a temperature-measuring resistor, and
one of the detection chambers (Sa) in the contact combustion-type gas sensor has a gas inlet that is provided with a silicone removal filter configured to adsorb and remove a silicone compound, to permit paraffinic hydrocarbon gas or hydrogen gas to transmit therethrough and to remove a solvent gas .

The target gas to be detected comprises a paraffinic hydrocarbon gas, a hydrogen gas, or a solvent gas. The gas detector of the present invention includes an output processing unit configured to acquire concentration data of a target gas being detected in a test gas on the basis of output data provided by the one gas detection element (20a) disposed in the one detection chamber (Sa) and to acquire concentration data of the target gas provided by the other gas detection element (20b) disposed in the other detection chamber (Sb) and to output the higher one of the two pieces of concentration data as the concentration indication value of the target gas being detected, wherein in the case of the target gas being a paraffinic hydrocarbon or hydrogen gas detection element (20a) indicates the higher output value and in the case of the target gas being a solvent gas detection element (20b) indicates the higher output value.

Furthermore, the gas detector of the present invention may preferably be configured such that the silicone removal filter is a filter including a support having air permeability, and silica carried by the support, the filter being subjected to an adsorption acceleration treatment by iron (III) chloride to accelerate adsorption of a silicone compound; or a filter including a support having air permeability and fumed silica carried by the support.

Still furthermore, the gas detector of the present invention may preferably include a sensor drive unit configured to drive the contact combustion-type gas sensor. The sensor drive unit may preferably intermittently drive each of the two gas detection elements so as to repeat the same or continuous energization duration and non-energization duration for each of the two gas detection elements.

Still furthermore, the sensor drive unit may preferably include a power source circuit that is common to the two gas detection elements and may preferably be configured to alternately energize each of the two gas detection elements.

The gas detector of the present invention may preferably be configured such that the energization duration is 0.5 to 2 seconds and the non-energization duration is one second or greater.

Furthermore, the gas detector of the present invention may preferably be configured such that each of the gas detection elements of the contact combustion-type gas sensor employs ZrO₂ or Al₂O₃ as the carrier and at least one type selected from the group consisting of Pt, Pd, PtO, PtO₂, and PdO as the catalyst.

Still furthermore, the gas detector of the present invention may preferably be configured such that the content ratio of the catalyst to the carrier is 10 to 30wt%.

### ADVANTAGEOUS EFFECTS OF INVENTION

According to the gas detector of the present invention, even when the gas detector is used in an environment where a silicone compound or a poisonous substance exists, it is possible to acquire highly reliable output for a target gas to be detected by at least one of the gas detection elements. Thus, the gas detector can be configured to have a high durability to silicone poisoning and is capable of accurately detecting the target gas to be detected.

Furthermore, because a high durability to silicone poisoning is provided, it is possible to reduce as much as possible the amount of carrier constituting the gas sensitive part in the gas detection element and thereby reduce the size of the gas detection element itself. It is thus possible to reduce the heat capacity of the gas detection element to thereby reduce power consumption.

Furthermore, the two gas detection elements may be intermittently driven, thereby enabling further reduction in power consumption.

### BRIEF DESCRIPTION OF DRAWINGS

[Fig. 1] illustrates an example structure of a contact combustion-type gas sensor to be used in a gas detector of the present invention; (a) an exploded perspective view, (b) a plan view with part of the structure not illustrated, and (c) a cross-sectional view.
[Fig. 2] is a cross-sectional view schematically illustrating the structure of an example of a gas detection element.
[Fig. 3] is a timing chart showing an example drive scheme of a contact combustion-type gas sensor.
[Fig. 4] is a conceptual diagram showing the tendency of output data from each gas detection element that is acquired for a reference gas of each of (a) a paraffinic hydrocarbon gas, (b) a solvent gas, and (c) a hydrogen gas.
[Fig. 5] is a conceptual diagram showing the tendency of output data from each gas detection element that is acquired for a reference gas of each of (a) the paraffinic hydrocarbon gas, (b) the solvent gas, and (c) the hydrogen gas when exposed for a predetermined period of time in an environment where a silicone compound exists.

### DESCRIPTION OF EMBODIMENTS

An embodiment of the present invention will be described in more detail below.

A gas detector of the present invention is provided with a contact combustion-type gas sensor which is designed for a target gas to be detected such as a paraffinic hydrocarbon gas, hydrogen gas, and a solvent gas.

The gas detector of the present invention may be configured either as a portable type or a stationary type; however, as will be discussed later, since the gas detector of the present invention can be configured as one of which power consumption has been reduced, the gas detector will be useful when configured as a portable type that operates on a battery.

The gas detector of the present invention is provided with a contact combustion-type gas sensor, a sensor drive unit configured to drive the contact combustion-type gas sensor, an output processing unit configured to process a gas detection signal from the contact combustion-type gas sensor, and a display unit.

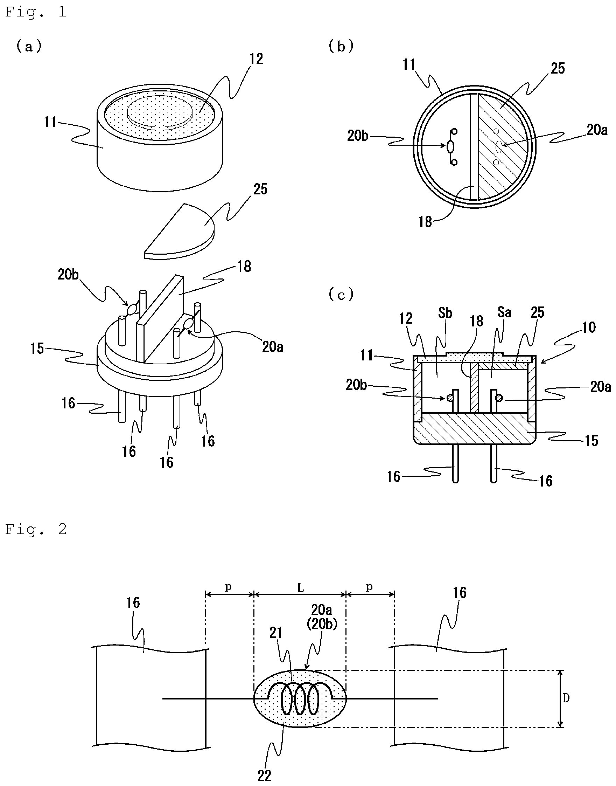

Fig. 1 illustrates an example structure of a contact combustion-type gas sensor to be used in a gas detector of the present invention; (a) an exploded perspective view, (b) a plan view with part of the structure not shown, and (c) a cross-sectional view.

The contact combustion-type gas sensor 10 is provided with a case 11 in which formed are two detection chambers Sa, Sb that are partitioned by a partitioning plate 18 serving also as a heat shielding plate; and two gas detection elements 20a, 20b which are disposed in the two detection chambers Sa, Sb, respectively.

The case 11 has one end side opening which is blocked by an anti-inflammatory filter 12 made of, for example, a metal sintered compact and which is, for example, cylindrical in shape, and has the other end side opening which is provided with a base member 15 for supporting the gas detection elements 20a, 20b so as to tightly block the other end side opening.

On one surface of the base member 15 is provided the flat partitioning plate 18 that divides the inner space of the case 11 into two halves, and the gas detection elements 20a, 20b are disposed on both sides that sandwich the partitioning plate 18, respectively. Each of the gas detection elements 20a, 20b has the ends secured to the top portions of leads 16, respectively, for example, in an attitude that extends horizontally along the partitioning plate 18. Each of the leads 16 is provided so as to tightly penetrate the base member 15 and protrude and extend outwardly in the axial direction.

As illustrated in Fig. 2, each of the gas detection elements 20a, 20b is configured from a temperature-measuring resistor 21 which is energized to generate heat and a gas sensitive part 22 which is firmly fixed to the temperature-measuring resistor 21.

The temperature-measuring resistor 21 is configured from a heater having a coil part into which a resistance wire having heat resistance and corrosion resistance is wound in a coil shape.

The temperature-measuring resistor 21 may be formed of, for example, platinum or an alloy thereof.

The gas sensitive part 22 is configured such that an oxidation catalyst is carried by a carrier made of a metal oxide sintered compact.

As examples of the metal oxides constituting the carrier, may be mentioned ZrO₂ (zirconia), Al₂O₃ (alumina), SiO₂ (silica), and zeolite.

As examples of the oxidation catalysts to be used, may be mentioned at least one type selected from the group consisting of Pt, Pd, PtO, PtO₂, and PdO.

The content ratio of the oxidation catalyst in the gas sensitive part 22 is, for example, 10 to 30wt%.

By way of example, the gas detection elements 20a, 20b may be configured in a manner such that the elemental wire diameter of the resistance wire constituting the temperature-measuring resistor 21 is φ 0.005 to φ 0.020 mm, the outer diameter of the coil part is 0.08 to 0.30 mm, the number of times of winding is 6 to 15 turns, and the length of the coil part is 0.10 to 0.40 mm.

The maximum outer diameter D of the gas sensitive part 22 is 0.10 to 0.50 mm, and the length L of the gas sensitive part 22 is 0.10 to 0.50 mm. Furthermore, the closest distance (pitch) p between the gas sensitive part 22 and the lead 16 is 0.10 to 0.50 mm.

Thus, in the contact combustion-type gas sensor 10, the gas inlet of the one detection chamber Sa is provided with a silicone removal filter 25 configured to adsorb and thereby remove a silicone compound.

For example, the silicone removal filter 25 is preferably employed by allowing a substrate having air permeability such as a pulp sheet to carry silica and being subjected to an adsorption acceleration treatment by iron (III) chloride to accelerate the adsorption of the silicone compound, or by allowing the substrate to carry fumed silica. This makes it possible to avoid the poisoning of the one gas detection element 20a by the silicone compound with reliability and, for example, permit a target gas being detected such as a paraffinic hydrocarbon gas or hydrogen gas to transmit therethrough. Note that some of target gases being detected, for example, a solvent gas is to be removed by the silicone removal filter 25 because the solvent gas has an adsorption property similar to that of the silicone compound.

For example, such a silicone removal filter 25 may be produced by employing the pulp sheet as a support and impregnating and drying a liquid material. As examples of the liquid materials, may be mentioned a dispersion liquid which is predominantly formed of silica with water as a solvent and contains an iron (iii) chloride hydrate. For example, the content ratio of the iron (iii) chloride hydrate is 0.3 to 3wt%. When the fumed silica is used as the silica, the liquid material does not need to include an iron (III) chloride hydrate.

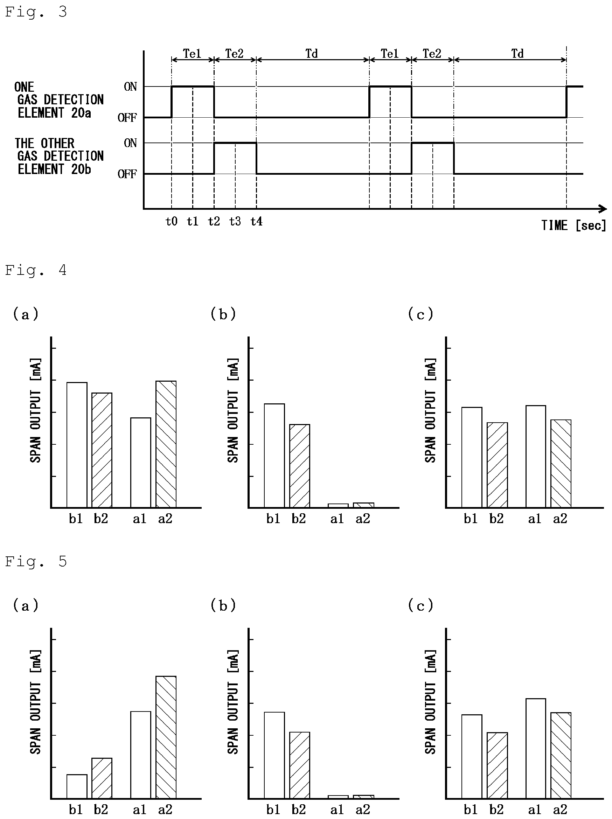

The sensor drive unit functions to intermittently drive each of the two gas detection elements 20a, 20b so as to repeat an energization duration and a non-energization duration of each of the two gas detection elements 20a, 20b. For example, as illustrated in Fig. 3, the sensor drive unit may preferably be configured to intermittently drive each of the gas detection elements 20a, 20b so as to alternately energize each of the two gas detection elements 20a, 20b and thereby repeat a gas detection cycle which consists of continuous energization durations Te1, Te2 and a non-energization duration Td for each of the two gas detection elements 20a, 20b. Note that the sensor drive unit may be configured to energize each of the two gas detection elements 20a, 20b at the same time, and the sequential order of energizing each of the gas detection elements 20a, 20b is not limited to a particular one.

As an operational condition of the contact combustion-type gas sensor 10, the voltage to be applied to each of the gas detection elements 20a, 20b is within the range, for example, from 0.50 to 1.20 V, more specifically, for example, 1.0 V. Furthermore, for example, the energization durations (energization time) Te1, Te2 of each of the gas detection elements 20a, 20b are, for example, 0.5 to 2 seconds, and may preferably be one second, for example. The non-energization duration (non-energization time) Td is, for example, one second or greater, and may preferably be 3 seconds, for example.

According to the drive scheme of such a contact combustion-type gas sensor 10, power consumption can be reduced, and due to a short de-energization time of the gas detection elements 20a, 20b, it is possible to acquire stable output without a warm-up process of the gas detection elements 20a, 20b for an extended period of time. In particular, when the two gas detection elements 20a, 20b are alternately energized, both the gas detection element 20a and the gas detection element 20b can be driven by a common power source circuit (not illustrated) . It is thus possible to reduce the power consumption of the gas detector.

The output processing unit functions to acquire concentration data of a target gas being detected in a test gas on the basis of output data on the test gas provided by the one gas detection element 20a, while acquiring concentration data of the target gas being detected in the test gas on the basis of output data on the test gas provided by the other gas detection element 20b. The output processing unit then functions to output the higher one of the two pieces of concentration data to a display unit as the concentration indication value of the target gas being detected.

More specifically, in the energization duration for one gas detection element, the output processing unit samples gas detection signals provided by the gas detection element, for example, at predetermined time intervals so as to sequentially acquire output data according to the gas detection element. Then, for example, on the basis of the temporally latest output data, the output processing unit acquires the concentration data of the target gas being detected in the test gas. Here, the output data according to the gas detection element is acquired, for example, at time intervals of 0.5 seconds.

Fig. 4 is a conceptual diagram illustrating the tendency of output data according to each of the gas detection elements 20a, 20b. Fig. 4(a) shows the tendency of output data on the paraffinic hydrocarbon gases of carbon numbers 1 to 6, (b) the tendency of output data on a solvent gas, and (c) the tendency of output data on a hydrogen gas.

In the example illustrated in Fig. 4, the output data of each of the two gas detection elements 20a, 20b is acquired by sampling gas detection signals provided by the gas detection element, for example, at time intervals of 0.5 seconds (at the points in time t1 to t4 in Fig. 3) for each of the two gas detection elements 20a, 20b in one energization duration Te1, Te2, for example, for one second when a contact combustion-type gas sensor is driven, for example, by the drive scheme illustrated in Fig. 3.

The vertical axes in Fig. 4(a) to (c) represent the span output value acquired by subtracting, from the output value provided by sampling the test gas, the output value provided by sampling introduced air in the same manner. Furthermore, a1 and a2 show output data according to the one gas detection element 20a disposed in one detection chamber Sa which is provided with the silicone removal filter 25, while b1 and b2 show output data according to the other gas detection element 20b disposed in the other detection chamber Sb which is not provided with the silicone removal filter 25. Then, the output data a2, b2 is the temporally latest data in the energization duration Te1, Te2 (shaded with diagonal lines for convenience sake) and used to acquire concentration data.

For example, as illustrated in Fig. 4 (a), since a paraffinic hydrocarbon gas such as methane transmits through the silicone removal filter 25, for each of the one gas detection element 20a and the other gas detection element 20b, the output data a1, a2, b1, and b2 having a span output value of a sufficiently high level is acquired. That is, the paraffinic hydrocarbon gas is detected by both the one gas detection element 20a and the other gas detection element 20b.

Furthermore, for example, as illustrated in Fig. 4(b), since a solvent gas such as an aromatic hydrocarbon like toluene, an alcohol, and a ketone is removed by the silicone removal filter 25, the span output values of the output data a1, a2 according to the one gas detection element 20a are substantially equal to "0," whereas as for the other gas detection element 20b, the output data b1, b2 having a span output value of a sufficiently high level is acquired. That is, the solvent gas is substantially not detected by the one gas detection element 20a but detected only by the other gas detection element 20b.

Still furthermore, as illustrated in Fig. 4(c), since the hydrogen gas transmits through the silicone removal filter 25, for each of the one gas detection element 20a and the other gas detection element 20b, the output data a1, a2, b1, b2 having a span output value of a sufficiently high level is acquired. That is, the hydrogen gas is detected by both the one gas detection element 20a and the other gas detection element 20b.

Then, in the illustrated example, for the paraffinic hydrocarbon gas, outputted to the display unit as the concentration indication value is the concentration data acquired on the basis of the output data (a2) according to the one gas detection element 20a which indicates a value higher than the concentration data acquired on the basis of the output data (b2) according to the other gas detection element 20b.

Likewise, for the solvent gas and the hydrogen gas, outputted to the display unit as the concentration indication value is the higher concentration data of the concentration data acquired on the basis of the output data (a2) of the one gas detection element 20a and the concentration data acquired on the basis of the output data (b2) of the other gas detection element 20b.

As described above, in the presence of a silicone compound or a poisonous substance in the atmosphere of the contact combustion-type gas sensor, the silicone compound is adsorbed and accumulated on the surface of an oxidation catalyst (poisoned), whereby the performance (reactivity) of the oxidation catalyst deteriorates to degrade the detection sensitivity.

However, the aforementioned gas detector is configured such that the gas inlet of the one detection chamber Sa is provided with the silicone removal filter 25. Thus, even when the gas detector is used in an environment in which a silicone compound exists, it is possible to acquire output with high reliability for a target gas being detected.

That is, as illustrated in Fig. 5(a), consider the paraffinic hydrocarbon gas which causes a significant output degradation level due to the poisoning of the gas detection element. In this case, there occurs a significant degradation in the span output values b1, b2 of the other gas detection element 20b disposed in the other detection chamber Sb where no silicone removal filter 25 is provided. However, for the one gas detection element 20a disposed in the one detection chamber Sa where the silicone removal filter 25 is provided, the output degradation level is extremely less significant because the silicone removal filter 25 adsorbs and removes the silicone compound. Thus, the one gas detection element 20a is capable of detecting the presence of the paraffinic hydrocarbon gas in the test gas.

Furthermore, as illustrated in Fig. 5(b), since the solvent gas causes a less significant output degradation level even when the gas detection element is poisoned (impervious to the influence of poisoning), there is a less significant output degradation level of the other gas detection element 20b that is disposed in the other detection chamber Sb where no silicone removal filter 25 is provided. Thus, while the one gas detection element 20a cannot detect the solvent gas because the solvent gas is removed by the silicone removal filter 25, the other gas detection element 20b is capable of detecting the presence of the solvent gas in the test gas.

Still furthermore, as illustrated in Fig. 5(c), since the hydrogen gas causes a less significant output degradation level even when the gas detection element is poisoned (impervious to the influence of poisoning) and transmits through the silicone removal filter 25, both the one gas detection element 20a and the other gas detection element 20b can detect the presence of the hydrogen gas in the test gas.

As described above, according to the aforementioned gas detector, even when the gas detector is used in an environment in which a silicone compound or a poisonous substance exists, at least one of the one gas detection element 20a and the other gas detection element 20b is capable of providing output data with sufficiently high reliability irrespective of the type of the target gas being detected. Thus, the gas detector can be configured as having a high durability to silicone poisoning. Then, it is possible to accurately detect the concentration of the target gas being detected on the basis of the acquired output data.

Furthermore, since the high durability to silicone poisoning is acquired, the amount of carrier constituting the gas sensitive part of the gas detection element can be reduced as much as possible, thereby allowing the gas detection element itself to be reduced in size. Thus, since the heat capacity of the gas detection element can be reduced, power consumption can be reduced.

Still furthermore, since each of the two gas detection elements 20a, 20b can be alternately energized, only one power source circuit is required to drive the two gas detection elements 20a, 20b. This configuration also makes it possible to reduce power consumption of the gas detector.

A description will now be given of example experiments that were performed in order to verify the effects of the present invention.

### [Example Experiment 1]

According to the structure illustrated in Fig. 1 and Fig. 2, a contact combustion-type gas sensor (A) was produced. The specifications of this contact combustion-type gas sensor (A) are shown as below.

### <Gas Detection Element>

Temperature-measuring resistor: Material, 10%Rh-90%Pt; Elemental wire diameter, φ 0.012 mm; Outer diameter of coil part, 0.18 mm; Number of turns, 8 turns; and Length of coil part, 0.20 mm
Carrier: Material, Sintered compact of Zirconia (75wt%) and Alumina (10wt%)
Oxidation catalyst: Material, Palladium, Content ratio: 14wt%
Maximum outer diameter D of gas sensitive part: 0.35 mm
Length L of gas sensitive part: 0.35 mm
Closest approach distance between gas sensitive part and lead (pitch) p: 0.3 mm

### <Silicone Removal Filter>

Material: Pulp sheet carrying silica and accelerated by iron (iii) chloride to adsorb a silicone compound
Thickness: about 1mm

A contact combustion-type gas sensor (B) having the same specification as that of the contact combustion-type gas sensor (A) was produced except that a filter including a pulp sheet and hydrophilic fumed silica ("AEROSIL 380" manufactured by Nippon Aerosil Co., Ltd.) carried by the pulp sheet was used as the silicone removal filter.

In each of the contact combustion-type gas sensors (A) and (B), the two gas detection elements were intermittently driven to repeat the gas detection cycles which continuously had the energization duration and the non-energization duration for each of the gas detection elements, and a test gas was acted thereon. Then, in one energization duration of each gas detection element, gas detection signals provided by the gas detection element were sampled, for example, at time intervals of 0.5 seconds to thereby acquire output data from each of the two gas detection elements. Then, on the basis of the temporally latest output data according to each gas detection element in one energization duration, concentration data was acquired. Table 1 below shows the concentration data representing higher values outputted as concentration indication values of the test gas.

Here, the applied voltage to the gas detection element was 1.0 V, the energization duration for the gas detection element was one second, and the non-energization duration was 3 seconds. Furthermore, used as the test gas was a methane gas with a concentration of 50 %LEL.

Then, each of the aforementioned contact combustion-type gas sensors (A) and (B) were subjected to a treatment with octamethylcyclotetrasiloxane (D4) with a concentration of 20 ppm for 20 minutes to thereby poison each of the contact combustion-type gas sensors (A) and (B). Then, in the same manner as described above, the concentration indication value of the test gas was acquired. The results are shown in Table 1 below.

### [Example Experiment 2]

In the same manner as in Example experiment 1 except that isopropyl alcohol (IPA) with a concentration of 50 %LEL was used as the test gas, the concentration indication value before poisoning and the concentration indication value after poisoning were acquired. The results are shown in Table 1 below.

### [Example Experiment 3]

In the same manner as in Example experiment 1 except that a hydrogen gas with a concentration 50%LEL was used as the test gas, the concentration indication value before poisoning and the concentration indication value after poisoning were acquired. The results are shown in Table 1 below.

**[Table 1]**

| | Combution-type Gas Sensor | Test Gas | | Concentration Ingication Value | |
|---|---|---|---|---|---|
| | | Type of Gas | Concentration | Before Poisining | After Poisining |
| Example 1 | Combution-type Gas Sensor (A) | Methane Gas | 50 %LEL | 50 %LEL | 50 %LEL |
| | Combution-type Gas Sensor (B) | | | 50 %LEL | 50 %LEL |
| Example 2 | Combution-type Gas Sensor (A) | IPA | 50 %LEL | 50 %LEL | 48 %LEL |
| | Combution-type Gas Sensor (B) | | | 50 %LEL | 46 %LEL |
| Example 3 | Combution-type Gas Sensor (A) | Hydrogen Gas | 50 %LEL | 50 %LEL | 50 %LEL |
| | Combution-type Gas Sensor (B) | | | 50 %LEL | 50 %LEL |

As is apparent from the results mentioned above, it was confirmed that even after poisoning, a target gas to be detected could be detected with certain accuracy irrespective of the type of the gas.

### REFERENCE SIGNS LIST

- 10: contact combustion-type gas sensor
- 11: case
- 12: anti-inflammatory filter
- 15: base member
- 16: lead
- 18: partitioning plate
- 20a: one gas detection element
- 20b: the other gas detection element
- 21: temperature-measuring resistor
- 22: gas sensitive part
- 25: silicone removal filter
- Sa: one detection chamber
- Sb: the other detection chamber

## Claims

1. A gas detector for detecting a target gas in a test gas comprising a contact combustion-type gas sensor (10) and an output processing unit, wherein:
the contact combustion-type gas sensor (10) includes a case (11) in which two detection chambers (Sa, Sb) are formed that are partitioned by a partitioning plate (18), and two gas detection elements (20a, 20b) are each disposed in each of two detection chambers (Sa, Sb) that are partitioned from each other, the gas detection elements (20a, 20b) each having a catalyst carried by a carrier made of a metal oxide sintered compact firmly fixed to a temperature-measuring resistor,
a gas inlet of the one detection chamber (Sa) is provided with a silicone removal filter (25) configured to adsorb and remove a silicone compound, to permit paraffinic hydrocarbon gas or hydrogen gas to transmit therethrough and to remove a solvent gas, and a gas inlet of the other detection chamber (Sb) is not provided with a silicone removal filter,
the target gas to be detected being a paraffinic hydrocarbon gas, a hydrogen gas or a solvent gas, and
the output processing unit is configured to acquire concentration data of a target gas being detected in a test gas on the basis of output data provided by the one gas detection element (20a) disposed in the one detection chamber (Sa) and to acquire concentration data of the target gas provided by the other gas detection element (20b) disposed in the other detection chamber (Sb) and to output the higher one of the two pieces of concentration data as the concentration indication value of the target gas being detected, wherein in the case of the target gas being a paraffinic hydrocarbon or hydrogen gas detection element (20a) indicates the higher output value and in the case of the target gas being a solvent gas detection element (20b) indicates the higher output value.

2. The gas detector according to claim 1, wherein the silicone removal filter (25) includes a support having air permeability and silica carried by the support and is further subjected to an adsorption acceleration treatment by iron (III) chloride to accelerate adsorption of a silicone compound.

3. The gas detector according to claim 1, wherein the silicone removal filter (25) includes a support having air permeability, and fumed silica carried by the support.

4. The gas detector according to any one of claims 1 to 3, comprising a sensor drive unit configured to drive the contact combustion-type gas sensor (10), wherein the sensor drive unit is configured to intermittently drive each of the two gas detection elements (20a, 20b) so as to repeat a gas detection cycle consisting of energization durations (Te1, Te2) and non-energization duration (Td), and to alternately energize or simultaneously energize each of the two gas detection elements (20a, 20b).

5. The gas detector according to claim 4, wherein the sensor drive unit includes a power source circuit that is common to the two gas detection elements (20a, 20b) and the sensor drive unit is configured to alternately energize each of the two gas detection elements (20a, 20b).

6. The gas detector according to claim 4 or 5, wherein the energization duration is 0.5 to 2 seconds and the non-energization duration is one second or greater.

7. The gas detector according to any one of claims 1 to 6, wherein each of the gas detection elements (20a, 20b) of the contact combustion-type gas sensor (10) employs any of ZrO₂ and Al₂O₃ as the carrier and at least one type selected from the group consisting of Pt, Pd, PtO, PtO₂, and PdO as the catalyst.

8. The gas detector according to claim 7, wherein a content ratio of the catalyst to the carrier is 10 to 30 wt%.

## Patentansprüche

1. Gasdetektor zum Detektieren eines Zielgases in einem Testgas, umfassend einen Gassensor (10) vom Kontaktverbrennungstyp und eine Ausgangsverarbeitungseinheit, wobei:
der Gassensor (10) vom Kontaktverbrennungstyp ein Gehäuse (11) enthält, in dem zwei Detektionskammern (Sa, Sb) ausgebildet sind, die durch eine Trennplatte (18) unterteilt sind, und zwei Gasdetektionselemente (20a, 20b) jeweils in zwei voneinander getrennten Detektionskammern (Sa, Sb) angeordnet sind, wobei die Gasdetektionselemente (20a, 20b) jeweils einen Katalysator aufweisen, der von einem Träger getragen wird, der aus einem Metalloxid-Sinterpressling besteht, der fest an einem Temperatur-Messwiderstand befestigt,
ein Gaseinlass der einen Detektionskammer (Sa) mit einem Silikonentfernungsfilter (25) versehen ist, der so konfiguriert ist, dass er eine Silikonverbindung adsorbiert und entfernt, um zu ermöglichen, dass paraffinisches Kohlenwasserstoffgas oder Wasserstoffgas hindurchtritt, und um ein Lösungsmittelgas zu entfernen, und ein Gaseinlass der anderen Detektionskammer (Sb) nicht mit einem Silikonentfernungsfilter versehen ist,
das zu detektierende Zielgas ein paraffinisches Kohlenwasserstoffgas, ein Wasserstoffgas oder ein Lösungsmittelgas ist, und
die Ausgabeverarbeitungseinheit derart konfiguriert ist, dass sie Konzentrationsdaten eines Zielgases, das in einem Testgas erfasst wird, auf der Grundlage von Ausgabedaten, die von dem einen Gasdetektionselement (20a) bereitgestellt werden, das in der einen Erfassungskammer (Sa) angeordnet ist, erfasst, und Konzentrationsdaten des Zielgases, welche von dem anderen Gasdetektionselement (20b) bereitgestellt werden, das in der anderen Detektionskammer (Sb) angeordnet ist, erfasst, und dass sie die höhere der zwei Konzentrationsdaten als den Konzentrationsanzeigewert des zu detektierenden Zielgases ausgibt, wobei in dem Fall, dass das Zielgas ein paraffinisches Kohlenwasserstoff- oder Wasserstoffgas ist, Detektionselement (20a) den höheren Ausgabewert anzeigt, und im Fall, dass das Zielgas ein Lösungsmittelgas ist, Detektionselement (20b) ist den höheren Ausgabewert anzeigt.

2. Gasdetektor nach Anspruch 1, wobei der Silikonentfernungsfilter (25) einen luftdurchlässigen Träger und von dem Träger getragene Silica enthält, und ferner einer Adsorptionsbeschleunigungsbehandlung durch Eisen(III)-Chlorid unterzogen wird, um die Adsorption einer Silikonverbindung zu beschleunigen.

3. Gasdetektor nach Anspruch 1, wobei der Silikonentfernungsfilter (25) einen luftdurchlässigen Träger und pyrogene Kieselsäure, die von dem Träger getragen wird, enthält.

4. Gasdetektor nach einem der Ansprüche 1 bis 3, umfassend eine Sensortreibereinheit, die derart konfiguriert ist, dass sie den Gassensor (10) vom Kontaktverbrennungstyp antreibt, wobei die Sensortreibereinheit so konfiguriert ist, dass sie jeden der beiden Gasdetektionselemente (20a, 20b) intermittierend antreibt, um einen Gasdetektionszyklus zu wiederholen, der aus Erregungsdauern (Te1, Te2) und Nichterregungsdauern (Td) besteht, und jedes der beiden Gasdetektionselemente (20a, 20b) abwechselnd zu erregen oder gleichzeitig zu erregen.

5. Gasdetektor nach Anspruch 4, wobei die Sensortreibereinheit eine Stromquellenschaltung umfasst, die an beide Gasdetektionselemente (20a, 20b) gekoppelt ist, und die Sensortreibereinheit so konfiguriert ist, dass sie abwechselnd jedes der beiden Gasdetektionselemente (20a, 20b) mit Energie versorgt.

6. Gasdetektor nach Anspruch 4 oder 5, wobei die Erregungsdauer 0,5 bis 2 Sekunden beträgt und die Nichterregungsdauer eine Sekunde oder mehr beträgt.

7. Gasdetektor nach einem der Ansprüche 1 bis 6, wobei jedes der Gasdetektionselemente (20a, 20b) des Gassensors (10) vom Kontaktverbrennungstyp ZrO₂ und Al₂O₃ als Träger und mindestens eine Komponente ausgewählt aus der Gruppe bestehend aus Pt, Pd, PtO, PtO₂ und PdO als Katalysator verwendet.

8. Gasdetektor nach Anspruch 7, wobei ein Anteilsverhältnis des Katalysators zu dem Träger 10 bis 30 Gew.-% beträgt.

## Revendications

1. Détecteur de gaz pour détecter un gaz cible dans un gaz test comprenant un capteur de gaz de type à combustion par contact (10) et une unité de traitement de sortie, dans lequel :
le capteur de gaz de type à combustion par contact (10) comprend un boîtier (11) dans lequel sont formées deux chambres de détection (Sa, Sb) qui sont séparées par une plaque de séparation (18), et deux éléments de détection de gaz (20a, 20b) sont disposés chacun dans chacune des deux chambres de détection (Sa, Sb) qui sont séparées l'une de l'autre, les éléments de détection de gaz (20a, 20b) ayant chacun un catalyseur porté par un support constitué d'un compact fritté d'oxyde métallique solidement fixé à une résistance de mesure de température,
une entrée de gaz de la première chambre de détection (Sa) est munie d'un filtre d'élimination de silicone (25) configuré pour adsorber et éliminer un composé de silicone, pour permettre à du gaz d'hydrocarbure paraffinique ou à du gaz hydrogène de passer à travers celui-ci et d'éliminer un gaz solvant, et une entrée de gaz de l'autre chambre de détection (Sb) n'est pas munie d'un filtre d'élimination de silicone, le gaz cible à détecter étant un gaz d'hydrocarbure paraffinique, un gaz hydrogène ou un gaz solvant, et
l'unité de traitement de sortie est configurée pour acquérir des données de concentration d'un gaz cible détecté dans un gaz test sur la base de données de sortie fournies par le premier élément de détection de gaz (20a) disposé dans la première chambre de détection (Sa) et pour acquérir des données de concentration du gaz cible fournies par l'autre élément de détection de gaz (20b) disposé dans l'autre chambre de détection (Sb) et pour délivrer en sortie le plus grand des deux morceaux de données de concentration en tant que valeur d'indication de concentration du gaz cible qui est détecté, dans lequel dans le cas où le gaz cible est un hydrocarbure paraffinique ou de l'hydrogène, l'élément de détection de gaz (20a) indique la valeur de sortie plus élevée et dans le cas où le gaz cible est un solvant, l'élément de détection de gaz (20b) indique la valeur de sortie plus élevée.

2. Détecteur de gaz selon la revendication 1, dans lequel le filtre d'élimination de silicone (25) comprend un support présentant une perméabilité à l'air et de la silice portée par le support et est en outre soumis à un traitement d'accélération d'adsorption par du chlorure de fer (III) pour accélérer l'adsorption d'un composé de silicone.

3. Détecteur de gaz selon la revendication 1, dans lequel le filtre d'élimination de silicone (25) comprend un support ayant une perméabilité à l'air, et de la silice fumée portée par le support.

4. Détecteur de gaz selon l'une quelconque des revendications 1 à 3, comprenant une unité d'entraînement de capteur configurée pour entraîner le capteur de gaz de type à combustion par contact (10), dans lequel l'unité d'entraînement de capteur est configurée pour entraîner par intermittence chacun des deux éléments de détection de gaz (20a, 20b) de façon à répéter un cycle de détection de gaz constitué de durées d'excitation (Te1, Te2) et d'une durée de non-excitation (Td), et pour exciter alternativement ou exciter simultanément chacun des deux éléments de détection de gaz (20a, 20b).

5. Détecteur de gaz selon la revendication 4, dans lequel l'unité d'entraînement de capteur comprend un circuit de source d'alimentation qui est commun aux deux éléments de détection de gaz (20a, 20b) et l'unité d'entraînement de capteur est configurée pour exciter alternativement chacun des deux éléments de détection de gaz (20a, 20b).

6. Détecteur de gaz selon la revendication 4 ou 5, dans lequel la durée d'excitation est de 0,5 à 2 secondes et la durée de non-excitation est d'une seconde ou plus.

7. Détecteur de gaz selon l'une quelconque des revendications 1 à 6, dans lequel chacun des éléments de détection de gaz (20a, 20b) du capteur de gaz de type à combustion par contact (10) utilise l'un quelconque parmi ZrO₂ et Al₂O₃ comme support et au moins un type choisi parmi le groupe constitué de Pt, Pd, PtO, PtO₂, et PdO en tant que catalyseur.

8. Détecteur de gaz selon la revendication 7, dans lequel un rapport de teneur entre le catalyseur et le support est de 10 à 30 % en poids.
